(19) Europäisches Patentamt
European Patent Office
Office européen des brevets

(11) EP 1 808 162 A1

(12) EUROPEAN PATENT APPLICATION

(43) Date of publication:
**18.07.2007 Bulletin 2007/29**

(51) Int Cl.:
***A61K 9/20*** *(2006.01)* ***A61K 31/4025*** *(2006.01)*
***A61K 31/40*** *(2006.01)*

(21) Application number: **05257590.9**

(22) Date of filing: **09.12.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **21.11.2005 US 738828 P**
**23.11.2005 WOPCT/US2005/042789**

(71) Applicant: **TEVA PHARMACEUTICAL INDUSTRIES LTD.**
**49131 Petah Tiqva (IL)**

(72) Inventors:
- **Dlugatch, Dafna**
  **Petach Tikva (IL)**
- **Doani, Zvika**
  **Tel Mond (IL)**

(74) Representative: **Gallagher, Kirk James et al**
**D Young & Co**
**120 Holborn**
**London EC1N 2DY (GB)**

# (54) Atorvastatin pharmaceutical formulation

(57) Provided are atorvastatin compositions which reduce the effect of food in the bioavailability of atorvastatin and methods for making such compositions. Also provided are methods of reducing low density lipoprotein by administering the compositions of the invention.

EP 1 808 162 A1

## Description

RELATED APPLICATIONS

This application claims the benefit of U.S. provisional application serial number AWAITED (attorney docket no. 1662/98401), filed November 21, 2005, the contents of which is incorporated herein by reference.

FIELD OF THE INVENTION

The invention encompasses atorvastatin compositions which reduce the effect of food on the bioavailability of atorvastatin, methods for making such compositions, and a method of reducing low density lipoprotein by administering the compositions of the invention.

BACKGROUND OF THE INVENTION

Atorvastatin has the chemical name [R-(R*, R*)]-2-(4-fluorophenyl)-β,δ-dihydroxy-5-(1-methylethyl)-3-phenyl-4-[(phenylamino)carbonyl]-1H-pyrrole-1-heptanoic acid and is depicted below in lactone form in formula (I) and its calcium salt of formula (II):

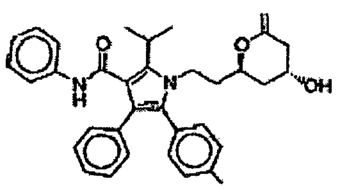

(I)

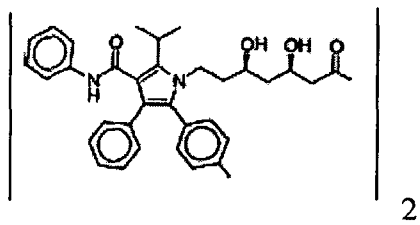

• $Ca^{2+}$

(II)

Atorvastatin is a member of the class of drugs called statins. Statin drugs are apparently currently the most therapeutically effective drugs available for reducing low density lipoprotein (LDL) particle concentration in the blood stream of subjects.

The mechanism of action of statin drugs has been elucidated in some detail. Statins supposedly interfere with the synthesis of cholesterol and other sterols in the liver by competitively inhibiting the 3-hydroxy-3-methyl-glutaryl-coelizyme, a reductase enzyme ("HMG-CoA reductase"). HMG-CoA reductase reportedly catalyzes the conversion HMG to mevalonate, which is the rate determining step in the biosynthesis of cholesterol.

Atorvastatin was supposedly disclosed in U.S. Patent No. 4,681,893. Atorvastatin hemi-calcium salt trihydrate is marketed under the name LIPITOR® by Pfizer, Inc.

In general, it is known that the absorption and bioavailability of any particular therapeutic agent can be affected by numerous factors when dosed orally. Such factors include the presence of food in the gastrointestinal (GI) tract because, in general, the gastric residence time of a drug is usually significantly longer in the presence of food than in the fasted state. If the bioavailability of a drug is affected beyond a certain point due to the presence of food in the GI tract, the drug is said to exhibit a "food effect."

Food effects are important inasmuch as, when a drug exhibits an adverse food effect, there is risk associated with administering it to a patient who has eaten recently. The risk derives from the potential that absorption into the bloodstream may be adversely affected to the point that the patient risks insufficient absorption to remediate the condition for which the drug was administered.

Atorvastatin is reported to have a very low absorption when taken with food as compared to when it is ingested alone. Therefore, one of the main challenges in the development of atorvastatin formulations is the effect of food on the bioavailability of atorvastatin. For example, bioavailability of atorvastatin may apparently be reduced by as much as 70% when it is ingested with food.

Therefore, there is a need for atorvastatin formulations and methods of their preparation that effectively reduce the food effect encountered by the administration of atorvastatin.

## SUMMARY OF THE INVENTION

The present invention provides atorvastatin formulations that effectively reduce the food effect associated with administration of atorvastatin.

In one embodiment, the present invention encompasses a pharmaceutical dosage form, which reduces food effect encountered by the administration of atorvastatin, comprising an effective amount of atorvastatin and a pharmaceutically acceptable excipient, wherein the dosage form exhibits a food effect of less than about 45% as characterized by $C_{max}$ values, and the atorvastatin contains at least one atorvastatin selected from the group consisting of atorvastatin hemi-calcium Form V, atorvastatin having an average particle size of at most about 50 microns, and micronized atorvastatin.

In another embodiment, the present invention encompasses a pharmaceutical dosage form, which reduces food effect encountered by the administration of atorvastatin, comprising an effective amount of atorvastatin and a pharmaceutically acceptable excipient, wherein the dosage form exhibits a food effect of less than about 45% as characterized by $C_{max}$ values, and the atorvastatin contains at least one atorvastatin selected from the group consisting of atorvastatin hemi-calcium Form V and micronized atorvastatin. Preferably, the micronized atorvastatin has a particle size of at most about 20 microns, and more preferably at most about 10 microns.

Preferably, the dosage form exhibits a food effect of less than about 30%, and more preferably less than about 20% as measured by $C_{max}$ values.

In a preferred embodiment, a dosage form of the invention provides a $C_{max\text{-}fed}$ of at least about 20 ng/ml when dosed at about 80 mg of atorvastatin. Preferably, the $C_{max\text{-}fed}$ is at least about 30 ng/ml, and more preferably at least about 40 ng/ml when dosed at about 80 mg of atorvastatin.

Preferably, the dosage form contains at least one of atorvastatin hemi-calcium Form V, atorvastatin hemi-calcium Form VIII having an average particle size of at most about 50 microns, micronized hemi-calcium Form V, or micronized hemi-calcium Form VIII. Also preferably, the dosage form contains at least one of atorvastatin hemi-calcium ethanolate (containing up to 3% ethanol), atorvastatin hemi-calcium iso-propanolate (containing up to 6% iso-propanol), or atorvastatin hemi-calcium hydrate.

In another preferred embodiment, the dosage form contains atorvastatin having an average particle size of at most about 20 microns, and more preferably, at most about 10 microns.

In one preferred embodiment, the excipient is at least one member selected from the group consisting of vitamin E, hydroxypropylcellulose, microcrystalline cellulose, crospovidone, sodium bicarbonate, meglumine, polacrilin, calcium phosphate, lactose, colloidal silicone dioxide, talc, magnesium stearate, croscarmellose, sodium carbonate, polyplasdone, magnesium aluminum silicate, sodium stearyl fumarate, and a coating.

In another preferred embodiment, the excipient is at least one member selected from the group consisting of mannitol, crospovidone, polyvinylpyrrolidone, vitamin E, tris hydroxymethylaminoethane, dibasic calcium phosphate anhydrous, sodium stearyl fumarate, and a coating.

In yet a further embodiment, the excipient is at least one member selected from the group consisting of calcium oxide, magnesium oxide, calcium magnesium carbonate, carbonates or bicarbonates of sodium, potassium or ammonium; ammonium or alkali metal salts of phosphoric acid or pyrophosphate; ammonium or alkali metal salts of carboxylic acids or fatty acids; calcium magnesium acetate, ammonium or alkali metal salts of aspartic or glutamic acid; carbonates of lysine or arginine; bicarbonates of lysine, arginine, cystine or histidine; free base forms of lysine, arginine, tryptophan, histidine, asparagine or glutamine; carboxylic acid salts of lysine, arginine or histidine; salt forms of cystine, phenols, biophenols or flavonoids, vitamin P, tyrosine, isoflavones, polymers carrying amine functions, polymers carrying acid functions in their salt forms, polyvinyl acetate or phthalate; and peptides or proteins with iso-electric point greater than 4.5.

Preferably, the dosage form is an oral dosage form, and more preferably in the form of a tablet.

The present invention also encompasses methods of preparing the dosage forms of the invention.

In one embodiment, the present invention encompasses a method of preparing a pharmaceutical dosage form, which reduces food effect encountered by the administration of atorvastatin, comprising preparing a mixture of atorvastatin and a pharmaceutically acceptable excipient, and formulating the mixture into a dosage form, wherein the dosage form exhibits a food effect of less than about 45% as characterized by $C_{max}$ values, and the atorvastatin contains at least one atorvastatin selected from the group consisting of atorvastatin hemi-calcium Form V, atorvastatin having an average particle size of at most about 50 microns, and micronized atorvastatin.

In one embodiment, the present invention encompasses a method of preparing a pharmaceutical dosage form, which reduces food effect encountered by the administration of atorvastatin, comprising preparing a mixture of atorvastatin and a pharmaceutically acceptable excipient, and formulating the mixture into a dosage form, wherein the dosage form exhibits a food effect of less than about 45% as characterized by $C_{max}$ values, and the atorvastatin contains at least one atorvastatin selected from the group consisting of atorvastatin hemi-calcium Form V, and micronized atorvastatin.

Preferably, the excipient is at least one member selected from the group consisting of vitamin E, hydroxypropylcellulose, microcrystalline cellulose, crospovidone, sodium bicarbonate, meglumine, polacrilin, calcium phosphate,

lactose, colloidal silicone dioxide, talc, magnesium stearate, croscarmellose, sodium carbonate, polyplasdone, magnesium aluminum silicate, sodium stearyl fumarate, and a coating.

Also preferably, the excipient is at least one member selected from the group consisting of mannitol, crospovidone, polyvinylpyrrolidone, vitamin E, tris hydroxymethylaminoethane, dibasic calcium phosphate anhydrous, sodium stearyl fumarate, and a coating.

The excipient is also preferably at least one member selected from the group consisting of calcium oxide, magnesium oxide, calcium magnesium carbonate, carbonates or bicarbonates of sodium, potassium or ammonium; ammonium or alkali metal salts of phosphoric acid or pyrophosphate; ammonium or alkali metal salts of carboxylic acids or fatty acids; calcium magnesium acetate, ammonium or alkali metal salts of aspartic or glutamic acid; carbonates of lysine or arginine; bicarbonates of lysine, arginine, cystine or histidine; free base forms of lysine, arginine, tryptophan, histidine, asparagine or glutamine; carboxylic acid salts of lysine, arginine or histidine; salt forms of cystine, phenols, biophenols or flavonoids, vitamin P, tyrosine, isoflavones, polymers carrying amine functions, polymers carrying acid functions in their salt forms, polyvinyl acetate or phthalate; and peptides or proteins with iso-electric point greater than 4.5.

In a preferred embodiment, the method of preparing a pharmaceutical dosage form, which reduces food effect encountered by the administration of atorvastatin, comprises preparing a mixture of atorvastatin and a pharmaceutically acceptable excipient; granulating the mixture to form granules; and formulating the granules into the dosage form.

In another preferred embodiment, the method of preparing a pharmaceutical dosage form, which reduces food effect encountered by the administration of atorvastatin, comprises preparing a mixture of atorvastatin and at least one pharmaceutically acceptable excipient; preparing a solution comprised of vitamin E and hydroxypropylcellulose; granulating the solution with the mixture to obtain granules; combining at least one of crospovidone or colloidal silicone dioxide with the granules; and adding at least one of magnesium stearate or talc to form the dosage form. Preferably, at least one member selected from the group consisting of microcrystalline cellulose, crospovidone, sodium bicarbonate, meglumine, polacrilin potassium, dibasic calcium phosphate anhydrous, and lactose monohydrate is added to the mixture with atorvastatin before granulation.

In a more preferred embodiment, the method of preparing a pharmaceutical dosage form, which reduces food effect encountered by the administration of atorvastatin, comprises preparing a mixture of atorvastatin hemi-calcium, microcrystalline cellulose, crospovidone, sodium bicarbonate, meglumine, polacrilin potassium, dibasic calcium phosphate anhydrous, and lactose monohydrate; preparing a solution comprised of vitamin E and hydroxypropylcellulose; granulating the solution with the mixture to obtain granules; mixing crospovidone and colloidal silicone dioxide with the granules; adding magnesium stearate and talc to form a granulate mixture; compressing the granulate mixture into a tablet; and coating the tablet to form the dosage form.

In a preferred embodiment, the dosage forms of the invention are formulated into an oral dosage form, preferably in tablet form, and more preferably in coated tablet form.

Another embodiment of the invention encompasses a method of reducing low density lipoprotein by administering an effective amount of the dosage forms of the invention to a patient in need thereof.

## DETAILED DESCRIPTION OF THE INVENTION

The invention encompasses pharmaceutical dosage forms which reduce the food effect encountered by the administration of atorvastatin, methods for their preparation, and methods of treatment using the same.

In one embodiment, the invention encompasses a pharmaceutical dosage form, which reduces food effect encountered by the administration of atorvastatin, comprising an effective amount of atorvastatin and a pharmaceutically acceptable excipient, wherein the dosage form exhibits a food effect as defined below of less than about 45%. Preferably, the food effect is less than about 30%, and more preferably less than about 20%.

As used herein, a "food effect" occurs when the maximum concentration in the blood plasma ($C_{max}$) of a drug in a subject is affected beyond a certain point due to the presence of food in the GI tract.

A food effect can be detected and quantified as described, for example, in Toothaker et al., ANN. REV. PHARMACOL. TOXICOL., vol. 20, 173-199, 1980, or in Welling et al., J. PHARM. SCI. vol. 68 (2), pp. 150-155, (1979). A food effect can be detected and quantified as described, for example, by determining the area under a curve (AUC), which plots the serum concentration (e.g., in µg/mL) of atorvastatin along the ordinate (Y-axis) against time along the abscissa (X-axis). Generally, the values for AUC represent a number of values taken from all the subjects in a patient test population and are, therefore, mean values averaged over the entire test population. By measuring the area under the curve for a fed population of subjects ($AUC_{fed}$) and comparing it with the area for the same population of fasted subjects ($AUC_{fast}$), it can be determined whether a given drug exhibits an adverse food effect or not. For example, food effect can be determined as follows:

$$\text{Food effect} = AUC_{fast} / AUC_{fed} \times 100.$$

Food effect can also be measured by determining the peak plasma concentration ($C_{max}$) of atorvastatin for a population of subjects. By determining the peak plasma concentration of atorvastatin for a population of fasted subjects ($C_{max-fast}$), and comparing it with the peak plasma concentration of atorvastatin for a population of fed subjects ($C_{max-fed}$), it can be determined whether a given drug exhibits an adverse food effect.
According to this invention, food effect is determined as follows:

$$\text{Food effect} = (C_{max-fast} - C_{max-fed}) / C_{max-fast} \times 100$$

As used herein, a food effect is considered "reduced" if it is less than about 45% as characterized by $C_{max}$ values.
In another embodiment, the dosage forms of the invention provide a $C_{max-fed}$ of at least about 20 ng/ml when dosed at about 80 mg of atorvastatin. Preferably, the $C_{max-fed}$ is at least about 30 ng/ml when dosed at about 80 mg of atorvastatin, and more preferably at least about 40 ng/ when dosed at about 80 mg of atorvastatin. One of ordinary skill in the art will appreciate that $C_{max}$ values will vary with the dose administered.
As used herein, a "population of fed subjects" is one made up of subjects each of whom has eaten a Food and Drug Administration (FDA)-recommended standard high fat breakfast within a period of twenty minutes, and then ingested (i.e., swallowed) the test dosage form essentially immediately thereafter. A standard high-fat breakfast consists of, for example, two eggs fried in one tablespoon of butter, two strips of bacon, six ounces of hash brown potatoes, two pieces of toast with two teaspoons of butter and two pats of jelly, and eight ounces of whole milk. This standard high-fat breakfast contains approximately 964 calories, 54% supplied as fat (58 gm) and 12% supplied as protein, calculated using the monograph "Nutritive Value of Foods," U.S. Department of Agriculture Home and Garden Bulletin No. 72. Additional food can also be consumed within the twenty minute period and the subject still qualifies as "fed." A "fasted subject" for purposes of definition and for measuring $AUC_{fast}$ is one who has not eaten for at least ten hours, typically overnight, prior to ingestion of the dosage form.
As used herein, the term "atorvastatin" includes atorvastatin, any anhydrate, hydrates, solvates, salts and equivalents thereof, and any crystalline or amorphous forms. Atorvastatin hemi-calcium is preferred. Also preferred are hydrate, iso-propanolate (containing up to 6% iso-propanol), and ethanolate (containing up to 3% ethanol) forms of atorvastatin.
The atorvastatin used in the dosage forms of the invention includes at least one of atorvastatin hemi-calcium Form V, or atorvastatin having an average particle size of at most about 50 $\mu$m. The atorvastatin used in the dosage forms of the invention can also include micronized atorvastatin.
Crystalline atorvastatin hemi-calcium Form V is disclosed in International Publication No. WO 01/36384, incorporated herein by reference in its entirety. Atorvastatin hemi-calcium Form V is preferably characterized by a powder X-ray diffraction pattern obtained using conventional $CuK_{\alpha}$ radiation having peaks at 5.3 $\pm$0.2 and 8.3 $\pm$0.2 degrees two-theta and a broad peak at 18-23 $\pm$0.2 degrees two-theta. Alternatively, atorvastatin hemi-calcium Form V may be characterized by a solid state $^{13}$C NMR spectrum having signals at 21.9, 25.9, 118.9, 122.5, 128.7, 161.0 and 167.1 ppm.
Crystalline atorvastatin hemi-calcium Form VIII is disclosed in International Publication No. WO 02/43732, also incorporated herein by reference in its entirety. Atorvastatin hemi-calcium Form VIII is preferably characterized by a powder X-ray diffraction pattern obtained using conventional $CuK_{\alpha}$ radiation having peaks at 9.3, 9.6, 16.3, 19.2, 20.0, 21.6, 22.4, 23.9$\pm$0.2 degrees two-theta. Further characteristic peaks may be found at 6.9, 17.1, 24.7, 25.6, and 26.5$\pm$0.2 degrees 2$\theta$.
Preferably, dosage forms of the invention comprising crystalline atorvastatin hemi-calcium Form V contain atorvastatin hemi-calcium Form V in an amount of at least about 50% by weight of the atorvastatin. The atorvastatin may be in micronized or non-micronized form.
Also preferably, dosage forms of the invention comprising crystalline atorvastatin hemi-calcium Form VIII contain atorvastatin hemi-calcium Form VIII in an amount of at least about 50% by weight of the atorvastatin. Atorvastatin hemi-calcium Form VIII is preferably in micronized form.
In a preferred embodiment, dosage forms of the invention contain atorvastatin having an average particle size of at most about 50 $\mu$m when measured across the longest axis, preferably at most about 20 $\mu$m, and more preferably at most about 10 $\mu$m. As used herein, the term "average particle size" means that at least 50% of the particles in a sample have the specified size.
In another preferred embodiment, dosage forms of the invention contain micronized atorvastatin. Generally, atorvastatin in micronized form has significant pharmaceutical advantages. The term "micronized atorvastatin" refers to

atorvastatin having a particle size distribution where at least about 90% of the particles have a particle size of at most about 50 microns when measured across the longest axis. Preferably, micronized atorvastatin has a particle size of at most about 20 microns, and more preferably at most about 10 microns.

Micronization may be a mechanical process that involves the application of force to a particle, thereby resulting in the break-up of the particle. Such force may be applied by collision of particles at high speeds. Micronization may be carried out, for example, by grinding, air-jet micronizer, Ball mill, or Pin mill to produce micronized particles.

The size of a particle is determined by any of the methods commonly known in the art. The following methods, for example, may be used: sieves, sedimentation, electrozone sensing (coulter counter), microscopy, or Low Angle Laser Light Scattering (LALLS). The preferred methods for the present invention are the methods most commonly used in the pharmaceutical industry, such as laser diffraction or sieve analysis

The dosage form of the invention may contain any pharmaceutically acceptable excipient known in the art.

In a preferred embodiment, the excipient is at least one of vitamin E, hydroxypropylcellulose, microcrystalline cellulose, crospovidone, sodium bicarbonate, meglumine, polacrilin including polacrilin potassium, calcium phosphate such as dibasic calcium phosphate anhydrous, lactose including the monohydrate, colloidal silicone dioxide, talc, magnesium stearate, croscarmellose, sodium carbonate, polyplasdone, magnesium aluminum silicate, sodium stearyl fumarate, or a coating such as Opadry.

In another preferred embodiment, the excipient is at least one of mannitol, crospovidone, polyvinylpyrrolidone, vitamin E, tris hydroxymethylaminoethane, dibasic calcium phosphate anhydrous, sodium stearyl fumarate, or a coating such as Opadry.

In a further embodiment, the excipient is at least one of calcium oxide, magnesium oxide, calcium magnesium carbonate, carbonates or bicarbonates of sodium, potassium or ammonium; ammonium or alkali metal salts of phosphoric acid or pyrophosphate; ammonium or alkali metal salts of carboxylic acids or fatty acids; calcium magnesium acetate, ammonium or alkali metal salts of aspartic or glutamic acid; carbonates of lysine or arginine; bicarbonates of lysine, arginine, cystine or histidine; free base forms of lysine, arginine, tryptophan, histidine, asparagine or glutamine; carboxylic acid salts of lysine, arginine or histidine; salt forms of cystine, phenols, biophenols or flavonoids, vitamin P, tyrosine, isoflavones, polymers carrying amine functions, polymers carrying acid functions in their salt forms, polyvinyl acetate or phthalate; and peptides or proteins with iso-electric point greater than 4.5.

The present invention also encompasses methods of preparing the pharmaceutical dosage forms of the invention.

In one embodiment, the present invention encompasses a method of preparing a pharmaceutical dosage form, which reduces the food effect encountered by the administration of atorvastatin, by preparing a mixture of atorvastatin and a pharmaceutically acceptable excipient, and formulating the mixture into a dosage form, wherein the dosage form exhibits a food effect of less than about 45% as characterized by $C_{max}$ values.

The atorvastatin contains at least one of atorvastatin hemi-calcium Form V atorvastatin having an average particle size of at most about 50 microns, or micronized atorvastatin. In another embodiment, the atorvastatin contains at least one of atorvastatin hemi-calcium Form V or micronized atorvastatin.

In one embodiment, the method of preparing the pharmaceutical dosage form of the invention includes preparing a mixture of atorvastatin and at least one of vitamin E, hydroxypropylcellulose, microcrystalline cellulose, crospovidone, sodium bicarbonate, meglumine, polacrilin including polacrilin potassium, calcium phosphate such as dibasic calcium phosphate anhydrous, lactose including the monohydrate, colloidal silicone dioxide, talc, magnesium stearate, croscarmellose, sodium carbonate, polyplasdone, magnesium aluminum silicate, sodium stearyl fumarate; and formulating the mixture into the dosage form.

In another embodiment, the method of preparing the pharmaceutical dosage form of the invention includes preparing a mixture of atorvastatin and at least one of mannitol, crospovidone, polyvinylpyrrolidone, vitamin E, tris hydroxymethylaminoethane, dibasic calcium phosphate anhydrous, sodium stearyl fumarate; and formulating the mixture into the dosage form.

One of ordinary skill in the art will appreciate that other excipients such as those exemplified herein may also be added to the mixture with atorvastatin.

Dosage forms of the invention may be prepared in accordance with customary processing techniques for pharmaceutical formulations wherein the ingredients are suitably processed and formulated into a dosage form, e.g., compressed into a tablet, with pharmaceutically acceptable excipients.

In a preferred embodiment, the method includes formulating the dosage form into an oral dosage form, such as a tablet. More preferably, the dosage form is formulated into a tablet and coated with a coating, such as Opadry.

In one embodiment, the method of preparing the pharmaceutical dosage form of the invention includes preparing a mixture of atorvastatin and a pharmaceutically acceptable excipient; granulating the mixture to form granules; and formulating the granules into the dosage form.

The term "granulation" refers to processes where granules are produced. Granulation may be carried out by any methods known in the art.

In one embodiment, the method of preparing the pharmaceutical dosage form of the invention includes preparing a mixture of atorvastatin and at least one pharmaceutically acceptable excipient; preparing a solution comprised of vitamin E and hydroxypropylcellulose; granulating the solution with the mixture to obtain granules; combining at least one of crospovidone or colloidal silicone dioxide with the granules; and adding at least one of magnesium stearate or talc to form the dosage form.

In a preferred embodiment, at least one of microcrystalline cellulose, crospovidone, sodium bicarbonate, meglumine, polacrilin, calcium phosphate, or lactose added into the mixture with atorvastatin before granulation. Preferably, all the ingredients are added into the mixture before granulation, and more preferably, in the order of atorvastatin calcium, microcrystalline cellulose, crospovidone, sodium bicarbonate, meglumine, polacrilin, calcium phosphate, and lactose.

In a more preferred embodiment, the present invention encompasses a method of preparing a pharmaceutical dosage form, which reduces the food effect encountered by the administration of atorvastatin, by preparing a mixture of atorvastatin and at least one of microcrystalline cellulose, crospovidone, sodium bicarbonate, meglumine, polacrilin potassium, dibasic calcium phosphate anhydrous, and lactose monohydrate; preparing a solution comprised of vitamin E and hydroxypropylcellulose; granulating the solution with the mixture to obtain granules; mixing crospovidone and colloidal silicone dioxide with the granules; adding magnesium stearate and talc to form a granulate mixture; compressing the granulate mixture into a tablet; and coating the tablet to form the dosage form.

Another embodiment of the invention encompasses a method of reducing low density lipoprotein by administering an effective amount of the dosage forms of the invention to a patient in need thereof.

As used herein, "effective amount" means an amount of atorvastatin that, when administered to a patient for treating a disease or other undesirable medical condition, is sufficient to have a beneficial effect with respect to that disease or condition. The "effective amount" will vary depending on the disease or condition and its severity, and the age, weight, etc., of the patient to be treated. Determining the effective amount of atorvastatin is within the ordinary skill of the art and requires no more than routine experimentation.

In a preferred embodiment, atorvastatin is present in an amount of from about 5% to about 20%, more preferably from about 5% to about 10%, and more preferably in an amount of about 8%

Preferred unit dosages of the pharmaceutical compositions of this invention typically contain from 0.5 to 100 mg of atorvastatin. More usually, the atorvastatin is present in a unit dosage in an amount of from 2.5 mg to 80 mg.

Dosage forms contemplated by the present invention may include diluents, such as cellulose-derived materials like powdered cellulose, microcrystalline cellulose, microfine cellulose, methyl cellulose, ethyl cellulose, hydroxyethyl cellulose, hydroxypropyl cellulose, hydroxypropylmethyl cellulose, carboxymethyl cellulose salts and other substituted and unsubstituted celluloses; starch; pregelatinized starch; inorganic diluents like calcium carbonate and calcium diphosphate and other diluents known to the pharmaceutical industry. Yet other suitable diluents include waxes, sugars and sugar alcohols like mannitol and sorbitol, acrylate polymers and copolymers, as well as pectin, dextrin and gelatin.

Further excipients that are within the contemplation of the present invention include binders, such as acacia gum, pregelatinized starch, sodium alginate, glucose and other binders used in wet and dry granulation and direct compression tableting processes. Excipients may further include disintegrants like sodium starch glycolate, crospovidone, low-substituted hydroxypropyl cellulose and others. In addition, excipients may include tableting lubricants like magnesium and calcium stearate and sodium stearyl fumarate; flavorings; sweeteners; preservatives; pharmacy parenteral (including subcutaneous, intramuscular, and intravenous), inhalant and ophthalmic administration. Although the most suitable route in any given case will depend on the nature and severity of the condition being treated, the most preferred route of the present invention is oral. Dosages may be conveniently presented in unit dosage form and prepared by any of the methods well-known in the art of pharmacy.

Dosage forms include solid dosage forms, like tablets, powders, capsules, suppositories, sachets, troches and losenges as well as liquid suspensions and elixirs.

Capsule dosages may be made of gelatin or other conventional encapsulating material. Tablets and powders may be coated. Tablets and powders may be coated with an enteric coating. The enteric coated powder forms may have coatings comprising phthalic acid cellulose acetate, hydroxypropylmethyl-cellulose phthalate, polyvinyl alcohol phthalate, carboxymethylethylcellulose, a copolymer of styrene and maleic acid, a copolymer of methacrylic acid and methyl methacrylate, and like materials, and if desired, they may be employed with suitable plasticizers and/or extending agents. A coated tablet may have a coating on the surface of the tablet or may be a tablet comprising a powder or granules with an enteric-coating.

Having described the invention with reference to certain preferred embodiments, other embodiments will become apparent to one skilled in the art from consideration of the specification. The invention is further defined by reference to the following examples describing in detail the preparation of the composition and methods of use of the invention. It will be apparent to those skilled in the art that many modifications, both to materials and methods, may be practiced without departing from the scope of the invention.

EXAMPLES

**Preparation of Atorvastatin Formulation in Example 9**

**Granulation Solution** - Vitamin E (TPGS) and hydroxypropylcellulose were dissolved into 95% ethanol.

**Part I** - The following materials were transferred into a Diosna high shear mixer in the following order: lactose monohydrate, croscarmellose sodium, atorvastatin hemi-calcium, meglumine sodium bicarbonate, , dibasic calcium phosphate anhydrous , magnesium aluminum metasilicate, polacrilin potassium, and microcrystalline cellulose. The material was mixed for 3 minutes, and dry granulated in a Glatt GPCG-15 fluid bed dryer until it reached 2.2% loss on drying. The resulting granules were sized through an oscillating granulator (Frewitt) equipped with 0.8 mm screen set at medium speed, and transferred into a dry blender.

**Part II** - Crospovidone was mixed with the granules for 10 minutes.

**Part III -** talc and magnesium stearate were added and mixed for 5 minutes, and the material was then compressed into 963 mg tablets.

The tablets were coated with a suspension of Opadry YS 1R 7003 H white in purified water to form coated tablets under the following conditions: inlet air temperature 55-65°C, outlet air temperature 38-44°C, spray rate 15-25 g/min, for a batch size of 2500 tablets.

Examples 1-8, 10 and 11 can be similarly prepared.

**Atorvastatin Formulation Bioavailability Results**

The following table illustrates the compositions and bioavailabilities of atorvastatin formulations prepared in Examples 1-11.

**Table 1. Atorvastatin formulations and bioavailability data for Examples 1-11**

| Example | 1 | 2 | 3 | 4 | 5 | 6 | 7 | 8 | 9 | 10 | 11 |
|---|---|---|---|---|---|---|---|---|---|---|---|
| # of subjects | 24 | 24 | 18 | 18 | 18 | 19 | 19 | 19 | 12 | 12 | 12 |
| Fast Cmax (ng/ml/hr) | 67.9 | 49.9 | 42.5 | 50.42 | 41.5 | 41.62 | 45.36 | 40.26 | 38.73 | 55.17 | 25.45 |
| Food Cmax (ng/ml/hr) | 20.7 | 16.5 | 21.17 | 19.34 | 18.64 | 20.85 | 20.6 | 21.35 | 26.24 | 37.17 | 19.9 |
| **Food effect (%)** | 69.5 | 66.9 | 50.3 | 61.6 | 55 | 49.9 | 54.6 | 47 | **32.2** | **32.6** | **21.8** |

| Ingredient | Percent Composition (w/w) | | | | | | | | | | |
|---|---|---|---|---|---|---|---|---|---|---|---|
| **PART I** | | | | | | | | | | | |
| Lactose mono. 100 | 16.8 | | 73.1 | 6.5 | 54.8 | | 6.5 | 6.5 | 6.5 | | |
| Mannitol | 17.2 | 32.6 | | | | 76 | | | | | 70.5 |
| Croscarmellos Na. | | | 4.1 | 8.7 | 4.2 | | 8.7 | 8.7 | 8.7 | | |
| Starch 1500 LM | 15.9 | | | | | | | | | | |
| Atorvastatin | 9 | 7.7 | 8.4 | 8.4 | 6.9 | 8.4 | 8.4 | 8.4 | 8.4 | | 8.4 |
| Meglumine | | | | 12.2 | | | 12.2 | 12.2 | 12.2 | | |
| Entery coat citric Acid | | | | | 18.3 | | | | | | |
| Na. Be Carbonate | | | | 18.3 | | | 18.3 | 18.3 | 18.3 | | |
| Eudragit E PO | | | 1.2 | | 1 | | | | | | |
| Di.Ba.Ca. Phosph. | | | | 8.8 | | | 8.8 | 8.8 | 8.8 | | 5 |
| Ca.Sulphate2 x $H_2O$ | | 36.1 | | | | | | | | | |
| Mag. Alum. Silicate | | | | | | 2.4 | | 1.5 | 1.5 | | |
| Polacrilin Potassium | | | 4.1 | 4.2 | 7.1 | | 4.2 | 4.2 | 4.2 | | |
| Avicel 101/102/112 | 22.5 | | | 15.2 | | | 15.2 | 13.7 | 13.7 | | |
| PVP K-30 | | | 3.3 | | 2.7 | 3.3 | | | | | 3.2 |
| **GRANULATION SOLUTION** | | | | | | | | | | | |
| Polysorbate 80 | 2 | 3.3 | | | | | | | | | |
| Vitamine E TPGS | | | 2.4 | 2.4 | 2 | 2.4 | 2.4 | 2.4 | 2.4 | | 2.4 |
| Klucel(hydroxyp ropylcellulose) | 3.2 | 2.3 | | 2.9 | | | 2.9 | 2.9 | 2.9 | | |
| Tris Hydroxymethyla minoethane | | | | | | | | | | | 1 |
| **PART II** | | | | | | | | | | | |
| Polyplasdone XL | | | | | | 4.1 | | | | | |
| Crospovidone NF | 8.7 | 3.7 | | 8.2 | | | 8.2 | 8.2 | 8.2 | | 4 |
| CrospovidoneX L-10 | | 9.6 | | | | | | | | | |
| **PART III** | | | | | | | | | | | |
| Na. Stear.Fumarate | 2.6 | 0.9 | 1 | | 1.1 | 1 | | | | | 1 |
| Talc | | | | 0.4 | | | 0.4 | 0.4 | 0.4 | | |
| Mg. Stearate | | | | 1.2 | | | 1.2 | 1.2 | 1.2 | | |
| **COATING** | | | | | | | | | | | |
| Opadry | 2.5 | 3.7 | 2.4 | 2.4 | 2 | 2.4 | 2.4 | 2.4 | 2.4 | | 2.9 |
| AUC fast (ng/ml/hr) | 124.9 | 103.9 | 121.4 | 107.8 | 98.95 | 119 | 113 | 116 | 150.9 | 180.8 | 141.5 |
| AUC fed (ng/ml/hr) | 174.9 | 144.7 | 149.1 | 150.7 | 142.4 | 157.6 | 165.8 | 146 | 143.5 | 150.5 | 131.7 |
| Disintegration Time | 0:06:21 | 0:01:42 | 0:04:50 | 0:07:43 | 0:03:59 | 0:05:58 | 0:05:29 | 0:04:37 | 0:05:53 | 0:17:10 | 0:15:58 |

Formulations in Examples 1-8 contain non-micronized API-grade atorvastatin hemi-calcium Form VIII. The formulation in Example 9 contains API-grade atorvastatin hemi-calcium Form VIII micronized by air jet mill. The micronized atorvastatin product contains 90% particles having a particle size of less than 10 microns, and 50% of particles less than 6 microns.

The formulation in Example 10 contains micronized API-grade atorvastatin hemi-calcium Form V. The formulation in Example 11 contains non-micronized API-grade atorvastatin hemi-calcium Form V.

As shown in Table 1, the food effect associated with administration of atorvastatin in the fed state is reduced using dosage forms of the invention illustrated in Examples 9, 10 and 11.

Subjects are fasted for 10 hours (overnight) at a dose of 80 mg. The average AUC for 12 to 18 volunteers in the fed state taking the dosage forms of the invention is greater compared to the average AUC for those taking the Lipitor® formulation in the fed state.

**Atorvastatin Formulation Dissolution Results**

The following table contains dissolution data for Examples 1-11 obtained using the Paddle method (USP type II) at 50 RPM and water, 900 ml at 37°C, as the dissolution medium.

| **Example** | **1** | **2** | **3** | **4** | **6** | **7** | **8** | **9** | **10** | **11** |
|---|---|---|---|---|---|---|---|---|---|---|
| % dissol. in $H_2O$, 5min | 15 | 45 | 22 | 22 | 44 | 32 | 32 | 38 | 22 | 15 |
| % dissol. in $H_2O$, 15min | 40 | 55 | 63 | 65 | 76 | 81 | 79 | 92 | 69 | 47 |
| % dissol. In $H_2O$, 30min | 85 | 60 | 79 | 90 | 83 | 100 | 89 | 99 | 97 | 65 |

**Claims**

1. A pharmaceutical dosage form, which reduces food effect encountered by administration of atorvastatin, comprising:

   (a) an effective amount of atorvastatin; and
   (b) a pharmaceutically acceptable excipient,

   wherein the dosage form exhibits a food effect of less than about 45% as **characterized by** $C_{max}$ values, and the atorvastatin contains at least one atorvastatin selected from the group consisting of atorvastatin hemi-calcium Form V, atorvastatin having an average particle size of at most about 50 microns, and micronized atorvastatin.

2. The pharmaceutical dosage form of claim 1 comprising at least one atorvastatin selected from the group consisting of atorvastatin hemi-calcium Form V and micronized atorvastatin.

3. The pharmaceutical dosage form of claim 2 comprising atorvastatin hemi-calcium Form V.

4. The pharmaceutical dosage form of claim 1 comprising at least one atorvastatin selected from the group consisting of atorvastatin hemi-calcium Form V having an average particle size of at most about 50 microns and micronized hemi-calcium Form V.

5. The pharmaceutical dosage form of claim 1 comprising at least one atorvastatin selected from the group consisting of atorvastatin hemi-calcium Form VIII having an average particle size of at most about 50 microns and micronized hemi-calcium Form VIII.

6. The pharmaceutical dosage form of claim 1 comprising at least one atorvastatin selected from the group consisting of atorvastatin hemi-calcium ethanolate, atorvastatin hemi-calcium iso-propanolate, and atorvastatin hemi-calcium hydrate.

7. The pharmaceutical dosage form of any preceding claim , wherein the dosage form exhibits a food effect of less than about 30%.

**8.** The pharmaceutical dosage form of claim 7, wherein the dosage form exhibits a food effect of less than about 20%.

**9.** The pharmaceutical dosage form of any preceding claim, wherein the dosage form exhibits a $C_{max\text{-}fed}$ of at least about 20 ng/ml when dosed at about 80 mg of atorvastatin.

**10.** The pharmaceutical dosage form of claim 9, wherein the dosage form exhibits a $C_{max\text{-}fed}$ of at least about 30 ng/ml when dosed at about 80 mg of atorvastatin.

**11.** The pharmaceutical dosage form of claim 10, wherein the dosage form exhibits a $C_{max\text{-}fed}$ of at least about 40 ng/ml when dosed at about 80 mg of atorvastatin.

**12.** The pharmaceutical dosage form of any preceding claim comprising atorvastatin having an average particle size of at most about 50 microns.

**13.** The pharmaceutical dosage form of claim 12 comprising atorvastatin having an average particle size of at most about 20 microns.

**14.** The pharmaceutical dosage form of claim 13 comprising atorvastatin having an average particle size of at most about 10 microns.

**15.** The pharmaceutical dosage form of any preceding claim , wherein the excipient is at least one member selected from the group consisting of vitamin E, hydroxypropylcellulose, microcrystalline cellulose, crospovidone, sodium bicarbonate, meglumine, polacrilin, calcium phosphate, lactose , colloidal silicone dioxide, talc, magnesium stearate, croscarmellose, sodium carbonate, polyplasdone, magnesium aluminum silicate, sodium stearyl fumarate, and a coating.

**16.** The pharmaceutical dosage form of any of claims 1 to 14, wherein the excipient is at least one member selected from the group consisting of mannitol, crospovidone, polyvinylpyrrolidone, vitamin E, tris hydroxymethylaminoethane, dibasic calcium phosphate anhydrous, sodium stearyl fumarate, and a coating.

**17.** The pharmaceutical dosage form of any of claims 1 to 14, wherein the excipient is at least one member selected from the group consisting of calcium oxide, magnesium oxide, calcium magnesium carbonate, carbonates or bicarbonates of sodium, potassium or ammonium; ammonium or alkali metal salts of phosphoric acid or pyrophosphate; ammonium or alkali metal salts of carboxylic acids or fatty acids; calcium magnesium acetate, ammonium or alkali metal salts of aspartic or glutamic acid; carbonates of lysine or arginine; bicarbonates of lysine, arginine, cystine or histidine; free base forms of lysine, arginine, tryptophan, histidine, asparagine or glutamine; carboxylic acid salts of lysine, arginine or histidine; salt forms of cystine, phenols, biophenols or flavonoids, vitamin P, tyrosine, isoflavones, polymers carrying amine functions, polymers carrying acid functions in their salt forms, polyvinyl acetate or phthalate; and peptides or proteins with iso-electric point greater than 4.5.

**18.** The pharmaceutical dosage form of any preceding claim in an oral dosage form.

**19.** The pharmaceutical dosage form of claim 18 in the form of a tablet.

**20.** A method of preparing a pharmaceutical dosage form of any preceding claim, comprising the steps of:

(a) preparing a mixture of atorvastatin and at least one pharmaceutically acceptable excipient; and
(b) formulating the mixture into a dosage form.

**21.** The method of claim 20, wherein the method comprises the steps of:

(a) preparing a mixture of atorvastatin and a pharmaceutically acceptable excipient;
(b) granulating the mixture to form granules; and
(c) formulating the granules into the dosage form.

**22.** The method of claim 20 or 21, wherein the method comprises the steps of:

(a) preparing a mixture of atorvastatin and at least one pharmaceutically acceptable excipient;

(b) preparing a solution comprised of vitamin E and hydroxypropylcellulose;
(c) granulating the mixture with the solution to obtain granules;
(d) combining at least one of crospovidone or colloidal silicone dioxide with the granules; and
(e) adding at least one of magnesium stearate or talc to form the dosage form.

**23.** The method of any of claims 20 to 22, further comprising adding to the mixture of step (a) at least one member selected from the group consisting of microcrystalline cellulose, crospovidone, sodium bicarbonate, meglumine, polacrilin potassium, dibasic calcium phosphate anhydrous, and lactose monohydrate.

**24.** The method of any of claims 20 to 23, wherein the mixture is formulated into an oral dosage form.

**25.** The method of any of claims 20 to 24, wherein the mixture is compressed into a tablet.

**26.** The method of claim 25, further comprising coating the tablet.

**27.** The method of any of claims 20 to 26, wherein the method comprises the steps of:

(a) preparing a mixture of atorvastatin hemi-calcium, microcrystalline cellulose, crospovidone, sodium bicarbonate, meglumine, polacrilin potassium, dibasic calcium phosphate anhydrous, and lactose monohydrate;
(b) preparing a solution comprised of vitamin E and hydroxypropylcellulose;
(c) granulating the mixture with the solution to obtain granules;
(d) mixing crospovidone and colloidal silicone dioxide with the granules;
(e) adding magnesium stearate and talc;
(f) compressing the resulting mixture into a tablet; and
(g) coating the tablet to form the dosage form.

**28.** A dosage form prepared according to the method of any of claims 20 to 27.

**29.** Use of a pharmaceutical dosage form of any of claims 1 to 19 or a pharmaceutical dosage form prepared according to the method of any of claims 20 to 28 for the manufacture of a medicament for use in reducing low density lipoprotein.

European Patent Office

# PARTIAL EUROPEAN SEARCH REPORT

which under Rule 45 of the European Patent Convention shall be considered, for the purposes of subsequent proceedings, as the European search report

Application Number
EP 05 25 7590

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 696 084 B2 (PACE GARY W ET AL) 24 February 2004 (2004-02-24)<br>* column 12, line 17 - line 27 *<br>----- | 1-21, 23-26, 28,29 | A61K9/20<br>A61K31/4025<br>A61K31/40 |

TECHNICAL FIELDS SEARCHED (IPC)

A61K

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

4

| Place of search | Date of completion of the search | Examiner |
|---|---|---|
| Munich | 23 March 2006 | Giacobbe, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C07)

European Patent Office

INCOMPLETE SEARCH SHEET C

Application Number
EP 05 25 7590

Claim(s) searched completely:
27

Claim(s) searched incompletely:
1-26, 28, 29

Reason for the limitation of the search:

Present claims 1-26, 28 and 29 relate to a pharmaceutical composition which has a
given desired property or effect, namely a particular food effect. According to the wording of the claims this effect should be provided by a not better specified excipient, however the description does not provide support and disclosure in the sense of Article 84 and 83 EPC for any correlation between the nature of the excipient and the desired effect. It is to be observed that Examples 1-9 contain a large number of "excipients" and nothing indicates if one of them is responsible for the effect, or some of them or even all of them together.

In view of the lack of correlation with any feature directly linked to the constitution of the product the skilled person can vary, when measuring the food effect, several parameters without restriction. For example, nothing hinders him from taking two commercial tablets of Lipitor(R) if one turns out to be insufficient for achieving the targeted Cmax. Therefore the functional feature appearing at the end of claim 1 cannot serve to delimit the product claimed. Consequently, claim 1 is directed to any composition comprising atorvastatin and at least one excipient.

The initial phase of the search of such subject-matter revealed a very large number of documents relevant tothe issue of novelty. So many documents were retrieved that it is impossible to determine which parts of the claims 1-26, 28 and 29 may be said to define subject-matter for which protection might legitimately be sought (Article 84 EPC). For these reasons, a meaningful search of the whole claimed subject matter of these claims could not be carried out (Rule 45 EPC). The extent of the search was consequently limited to the subject-matter of claim 27, reflecting examples 1-9.

## ANNEX TO THE EUROPEAN SEARCH REPORT ON EUROPEAN PATENT APPLICATION NO. EP 05 25 7590

This annex lists the patent family members relating to the patent documents cited in the above-mentioned European search report.
The members are as contained in the European Patent Office EDP file on
The European Patent Office is in no way liable for these particulars which are merely given for the purpose of information.

23-03-2006

| Patent document cited in search report | Publication date | Patent family member(s) | Publication date |
|---|---|---|---|
| US 6696084 B2 | 24-02-2004 | US 2002056206 A1 | 16-05-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4681893 A
- WO 0136384 A
- WO 0243732 A

### Non-patent literature cited in the description


- **TOOTHAKER et al.** *ANN. REV. PHARMACOL. TOXICOL.,* 1980, vol. 20, 173-199
- **WELLING et al.** *J. PHARM. SCI.,* 1979, vol. 68 (2), 150-155